Europäisches Patentamt

European Patent Office

Office européen des brevets

(11) Publication number: **0 236 416 B1**

# EUROPEAN PATENT SPECIFICATION

(45) Date of publication of patent specification: **29.07.92**

(51) Int. Cl.⁵: **C12P 21/02**, C07K 1/06, C07K 7/36, C12N 9/02

(21) Application number: **86905378.5**

(22) Date of filing: **19.09.86**

(86) International application number: **PCT/GB86/00559**

(87) International publication number: **WO 87/01729 (26.03.87 87/07)**

(54) **POLYPEPTIDE PRODUCTION.**

(30) Priority: **19.09.85 GB 8523156**

(43) Date of publication of application: **16.09.87 Bulletin 87/38**

(45) Publication of the grant of the patent: **29.07.92 Bulletin 92/31**

(84) Designated Contracting States: **AT BE CH DE FR GB IT LI LU NL SE**

(56) References cited:
**EP-A- 0 133 282
EP-A- 0 134 631**

**Nature vol. 298 12.8.82 Bradbury et al "Mechanism of C-terminal amide formation...."pp 686-688**

**FEBS letters vol. 152 no. 2 Feb. 1983 Husain et al "Formation of the COOH terminal amide group....."**

**See also references of WO8701729**

(73) Proprietor: **CELLTECH LIMITED 244-250 Bath Road Slough Berkshire SL1 4DY(GB)**

(72) Inventor: **EATON, Michael, Anthony, William "Nethercote" Chinnor Road Aston Rowant, Oxfordshire OX9 5SH(GB)**
Inventor: **TITMAS, Richard, Charles, Dominic 26 Prince Andrew Close Boulter's Lock Maidenhead, Berkshire SL6 8OR(GB)**
Inventor: **RHIND, Stephen, Keith 47 Stratford Drive Wooburn Buckinghamshire HP10 0OO(GB)**

(74) Representative: **Votier, Sidney David et al CARPMAELS & RANSFORD 43, Bloomsbury Square London WC1A 2RA(GB)**

## Description

### Field of the Invention

This invention relates to a process for producing a polypeptide having a C-terminal amide group, where the polypeptide has one or more cysteine or cystine amino acid residues, comprising the step of reacting a polypeptide comprising the amino acid sequence of the polypeptide and a C-terminal glycine amino acid residue, with an amidating enzyme.

### Background to the Invention

A number of physiologically active polypeptides, especially peptide hormones, have been shown to depend upon a C-terminal amide group for their full activity. In addition, the C-terminal amide has been shown to enhance the half-life of some hormones in the circulatory system by preventing degradation from the C-terminal end by natural proteases. It is now possible, using the techniques of recombinant DNA technology, to synthesise polypeptides on an industrial scale by in vitro culture. However, polypeptides directly produced in this manner do not possess a C-terminal amide and may not therefore exhibit their full physiological activity and stability in the circulatory system. This problem has been overcome by the use of an amidating enzyme to convert an additional C-terminal amino acid residue into the necessary amide group (see for example published European Patent Application EP-A1-0134631 and EP-A1-0133282). The amidating enzyme concerned was first described and characterised by Bradbury et. al. (Bradbury, A. F. et. al. Nature (1982) 298, 686-688; Bradbury, A. F. et. al. Biochem. and Biophys. Comm. (1983) 1123(2), 372-377; Landymore-Lin, A. E. N. et. al. Biochem. And Biophys. Comm. (1983) 117(2), 289-293).

We have now discovered that such amidating enzymes do not satisfactorily amidate certain polypeptides, specifically those polypeptides which contain cysteine amino acid residues or disulphide bridges in their structure. Our research indicates that the lack of satisfactory amidation is due to precipitation of the polypeptide substrate during treatment with the amidating enzyme.

Without prejudice it is believed that this precipitation may be caused, at least in part, by copper which in some cases has been shown to enhance the activity of the amidating enzyme. The copper present in the amidating enzyme preparation may interact with cysteine or cystine sulphur groups giving rise to polymerisation of the polypeptide, leading to dimer formation and/or aggregation, which in turn may result in precipitation of the polypeptide. In addition, ascorbic acid (Vitamin C), which is used as a further cofactor in the amidation preparation, may cause further complications. For instance, ascorbic acid may also be involved in modification of the polypeptide as a result of its interaction with cysteine or cystine sulphur groups.

It appears that the amidating activity of the enzyme is considerably reduced when the polypeptide substrate is in a precipitated form.

### Summary of the Invention

According to a first aspect of the invention there is provided a process for producing a first polypeptide having a C-terminal amide group, where the first polypeptide has one or more cysteine or cystine amino acid residues, comprising the step of reacting a second polypeptide comprising the amino acid sequence of the first polypeptide and a C-terminal glycine amino acid residue , with an amidating enzyme, characterised in that the cysteine or cystine sulphur group or groups is or are protected at least during the amidation step.

As used herein the term 'amidating enzyme' is used to denote an enzyme which is capable of converting the C-terminal glycine amino acid residue to an amide group.

The amidating enzyme may be derived from sources such as for example mammalian tissue or from any other suitable source.

In a particularly preferred embodiment the amidating enzyme used in the process of the invention is an $\alpha$ amidating enzyme such as the $\alpha$ amidating enzyme derived from porcine pituitary tissue which was described and characterised by Bradbury et al (J. loc. cit.).

The protection of the cysteine or cystine sulphur group or groups during treatment with the amidating enzyme preparation reduces the hitherto unforeseen problem of precipitation.

The term "cysteine or cystine amino acid residues" as used herein includes derivatives thereof. Typically the cysteine or cystine group is capable of chemical modification either by derivatisation or reaction with a protecting group to yield a protected sulphur containing group.

The protection of the cysteine or cystine sulphur group employed during the amidation step may comprise irreversible protection. For instance, a cysteine or cystine sulphur group may be protected by irreversible derivatisation; for example, treatment with acrylonitrile, ethyleneimine, sulphenyl halide, or performic acid oxidation of cystine to cysteic acid. Preferably, however, the protection is reversible, and any protecting group capable of reversibly binding to the cysteine or cystine may be employed. Typically the conditions employed for protection of the cysteine or cystine sulphur group and, in the case of reversible protection, for removal of the protecting group are such that they do not have a detrimental effect on the structure of the polypeptide i.e. advantageously the final product is produced in the form of its native three-dimensional structure.

The polypeptide may be any polypeptide containing one or more cysteine or cystine amino acid residues for which a C-terminal amide group may be desirable. The process is suitable for the conversion of a C-terminal glycine amino acid residue of a polypeptide produced by recombinant DNA techniques to a C-terminal amide group. Examples of such polypeptides include calcitonins such as salmon calcitonin, human calcitonin and functionally equivalent derivatives of these calcitonins; calcitonin gene-related peptides such as human calcitonin gene-related peptide, rat calcitonin gene-related peptide and functionally equivalent derivatives of these gene-related peptides; oxytocin and functionally equivalent derivatives; and vasopressin and functionally equivalent derivatives.

The term 'polypeptide' as used herein denotes a compound comprising two or more amino acid residues and includes proteins having secondary structure.

In some cases under the conditions which are optimal for activity of the amidating enzyme (for example at a pH in the range 5 to 8, preferably about 6.8 for the preferred amidating enzyme ), the polypeptide-glycine compound may be close to, or at, its isoelectric point and this may cause precipitation, reducing, in another way, the substrate available for the amidating enzyme. This problem may be overcome by using protection which introduces a net charge into the polypeptide, either by derivatisation or use of a charged protecting group. The introduction of a net charge into the protected polypeptide may modify its isoelectric point and solubility properties and thereby reduce precipitation. Suitable protecting groups which may be bound to the cysteine or cystine sulphur atoms include $-SO_3^-$ and $-SPO_3^{2-}$ . Since human calcitonin-glycine and human calcitonin gene related peptide-glycine are close to their isoelectric point at the preferred amidation pH, the use of a charged protecting group, protecting the disulphide bridge, and altering the isoelectric point provides a significant improvement in the preparation of correctly C-terminal amidated polypeptides.

In a second aspect the invention provides a polypeptide having one or more cysteine or cystine amino acid residues and a C-terminal glycine amino acid residue characterised in that the or each cysteine or cystine sulphur group or groups is or are protected.

Each cysteine or cystine sulphur group may be protected by derivatisation or by binding of a protecting group. Suitable protecting groups include any protecting group capable of reversibly binding to the cysteine or cystine sulphur atom providing that it may be bound and removed under conditions which are not detrimental to the polypeptide. Preferably the protecting group is charged. Particularly preferred protecting groups are $-SO_3^-$ and $-SPO_3^{2-}$ .

Examples of other suitable cysteine or cystine sulphur protecting groups which may be used include for example benzyl groups, which may be substituted by one or more carboxyl, sulphate, phosphate, alkyl, alkoxy, nitro or halogen substituents e.g. a dinitrofluorobenzene group; a diphenylmethyl group, which may be substituted by one or more carboxyl, sulphate or phosphate substituents; a triphenylmethyl group which may be substituted by one or more carboxyl, sulphate or phosphate substituents; alkyl groups, e.g.a t-butyl group; an acyl group e.g. an acetyl group; an acyl group substituted by for example a halogen atom e.g. an iodoacetate group; a benzoyl group; a carbonate group; a carbamoyl group which may be substituted by for example an alkyl, an alkoxy or an alkoxyalkyl group; a benzhydryl group; a mono or di acetal group, e.g. a tetrahydropyranyl group or a benzylthiomethyl group; a metal e.g. mercury; a mercurial group, e.g. a mercuribenzoate group; or a mixed disulphide group e.g. an S-alkylmercapto or an S-alkylsulfenylcysteine group. For examples of other cysteine or cystine sulphur protecting groups and for methods of removing such protecting groups, if so desired, see for example "The Peptides: Analysis, Synthesis, Biology", Vol. 2 Part A ( eds. Gross and Meienhofer, Academic Press (1980) ) and "Chemical Modification of Proteins" ( eds. Means and Feeney, Holden-Day Inc. (1971) ).

It will be appreciated that a net charge may, if desired, be introduced into the above cysteine or cystine sulphur protecting groups, or any other suitable cysteine or cystine sulphur protecting group, by substitution of the protecting group with an appropriate charged substituent for example by substitution by one or more carboxyl, sulphate or phosphate substituents.

We have discovered also that if the copper which is present in the amidating enzyme preparation is

EP 0 236 416 B1

present in a chelated form, improved yields of amidated product may be obtained. Chelation of copper may be used as an alternative or preferably in combination with protection of cysteine or cystine sulphur groups. It appears that chelation of the copper may diminish precipitation of the polypeptide, and may have further advantages in terms of modulating accessability of copper to the enzyme. In this latter respect, we have further discovered that whilst in some cases copper enhances the activity of the amidating enzyme, excess copper can be detrimental to the enzyme activity. Chelation of the copper appears to overcome this difficulty.

Thus, in a third aspect the invention provides a process for producing a first polypeptide having a C-terminal amide group, comprising the step of reacting a second polypeptide comprising the amino acid sequence of the first polypeptide and a C-terminal glycine amino acid residue, with an amidating enzyme preparation, characterised in that the amidating enzyme preparation includes a compound capable of chelating copper.

Suitable chelating compounds include ethylenediaminetetracetic acid (EDTA) and penicillamine. A further chelating compound that may be used is bathocuproine, as a disulphonate, which especially chelates copper (I).

We have discovered that the addition of a chelating agent reduces the level of free copper, thereby reducing detrimental effects on both the polypeptide and the amidating enzyme but maintains a level of copper, in a suitable form, sufficient to act as a cofactor for the enzyme.

Brief Description of the Drawings

The invention is illustrated by the following examples with reference to the accompanying drawings which are high performance liquid chromatography (HPLC) traces.

Figure 1: Trace 1 shows an HPLC trace of an aliquot of a reaction mixture containing hCT-gly and porcine pituitary amidating enzyme taken at Omin incubation.

Trace 2 shows an HPLC trace of an aliquot of a reaction mixture containing hCT-gly and porcine pituitary amidating enzyme taken after incubation for 420min.

Figure 2: Trace 1 shows an HPLC trace of an aliquot of a pellet obtained after centrifugation of a reaction mixture incubated for 540min containing hCT-gly and porcine pituitary amidating enzyme

Trace 2 shows an HPLC trace of an aliquot of a supernatant obtained after centrifugation of a reaction mixture incubated for 540min containing hCT-gly and porcine pituitary amidating enzyme

Figure 3: Trace 1 shows an HPLC trace of a sample taken immediately after preparation of a reaction mixture containing hCT-gly, Cu/EDTA and ascorbic acid.

Trace 2 shows an HPLC trace of a sample taken after overnight incubation of a reaction mixture containing hCT-gly in the absence of ascorbic acid and Cu/EDTA

Trace 3 shows an HPLC trace of a sample taken after overnight incubation of a reaction mixture containing hCT-gly, Cu/EDTA and ascorbic acid

Figure 4: shows a trace run at increased sensitivity of Trace 3 shown in Figure 3.

Figure 5: Trace 1 shows an HPLC trace of an aliquot taken after incubation for 60min of a reaction mixture containing hCT and $Na_2SO_3$ .

Trace 2 shows an HPLC trace of an aliquot taken after incubation for 60min of a reaction mixture containing hCT-gly and $Na_2SO_3$ .

Figure 6: Trace 1 shows an HPLC trace of an aliquot taken immediately after preparation of a reaction mixture containing S-sulphonated hCT-gly and porcine amidating enzyme preparation .

Trace 2 shows an HPLC trace of an aliquot taken after incubation for 4hr of a reaction mixture containing S-sulphonated hCT-gly and porcine amidating enzyme preparation .

Figure 7: Trace 1 shows an HPLC trace of an aliquot of a reaction mixture containing hCT-gly, Cu/EDTA and $Na_3O_3PS$ taken after a reaction time of 30min.

Trace 2 shows an HPLC trace of an aliquot of a reaction mixture containing hCT-gly, Cu/EDTA and $Na_3O_3PS$ taken after a reaction time of 90min.

Trace 3 shows an HPLC trace of an aliquot of a reaction mixture containing hCT-gly, Cu/EDTA and $Na_3O_3PS$ taken after a reaction time of 150min.

Figure 8: Trace 1 shows an HPLC trace of an aliquot taken immediately after preparation of a reaction mixture containing hCT-gly and $Na_3O_3PS$.

Trace 2 shows an HPLC trace of an aliquot of a reaction mixture containing hCT-gly and $Na_3O_3PS$ taken after a reaction time of 30min.

Trace 3 shows an HPLC trace of an aliquot of a reaction mixture containing hCT-gly and $Na_3O_3PS$ taken after a reaction time of 60min.

4

Trace 4 shows an HPLC trace of an aliquot of a control reaction mixture

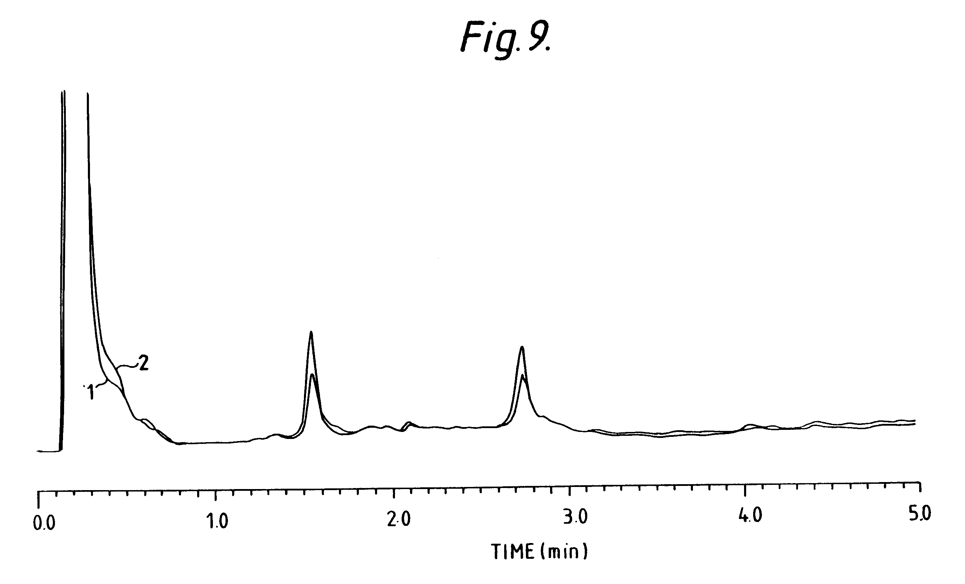

Figure 9: Trace 1 shows an HPLC trace of an aliquot taken immediately after preparation of a reaction mixture containing hCT-gly and $Na_3O_3PS$.

Trace 2 shows an HPLC trace of an aliquot of a reaction mixture containing hCT-gly and $Na_3O_3PS$ taken after a reaction time of 30min.

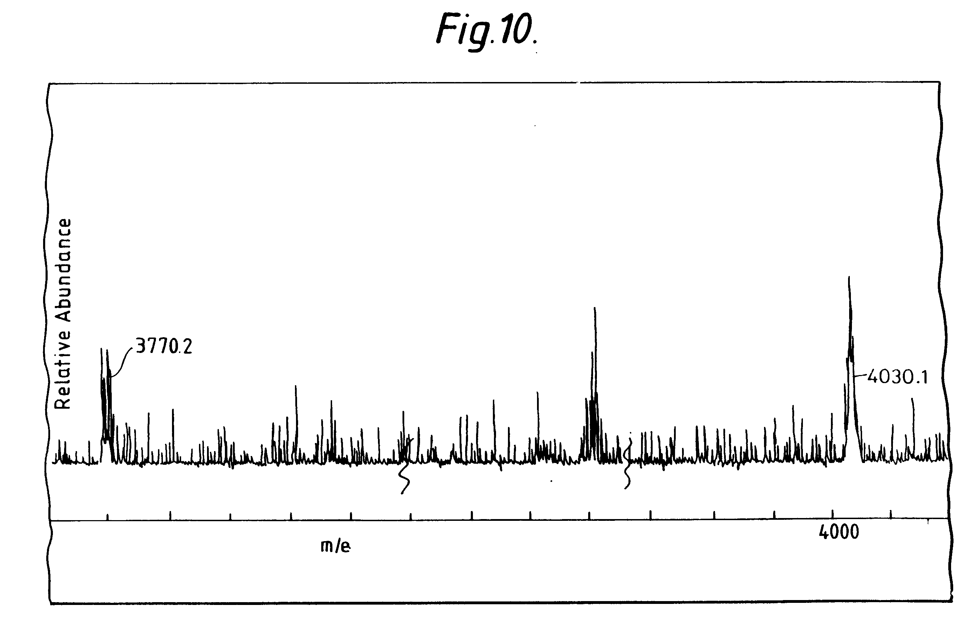

Figure 10: shows a mass spectrum of a sample of sulphonated hCGRP.

Detailed Description of the Embodiments

Human calcitonin is a peptide hormone with possible clinical applications in the treatment of post menopausal osteoporosis and other disorders of calcium metabolism, for example Paget's disease.

A precursor to this, human calcitonyl-glycine (hCT-gly) has been made in E. coli by recombinant DNA technology (see for example published European Patent Application EP-A1-0131363). The purified precursor can be amidated under selective conditions using an amidating enzyme as described in the above introduction.

Comparative Example A

An experiment was conducted to illustrate loss of human calcitonyl-glycine substrate during an amidation reaction.

Human calcitonyl-glycine was incubated at 37°C with an amidating enzyme. The composition of the reaction mixture (210 ul) was as follows:

25mM PIPES buffer (pH 6.8)
300 mM NaCl
100 uM Cu/EDTA
1 mM ascorbic acid
2 mg/ml human calcitonyl-glycine
1000 units/ml Catalase
200 ul porcine pituitary amidating enzyme

(The porcine pituitary amidating enzyme used was partially purified according to the method of: Bradbury, A. F., and Smyth, D.G. (1985) in "Biogenetics of Neurohormonal Peptides" pp 171-186).

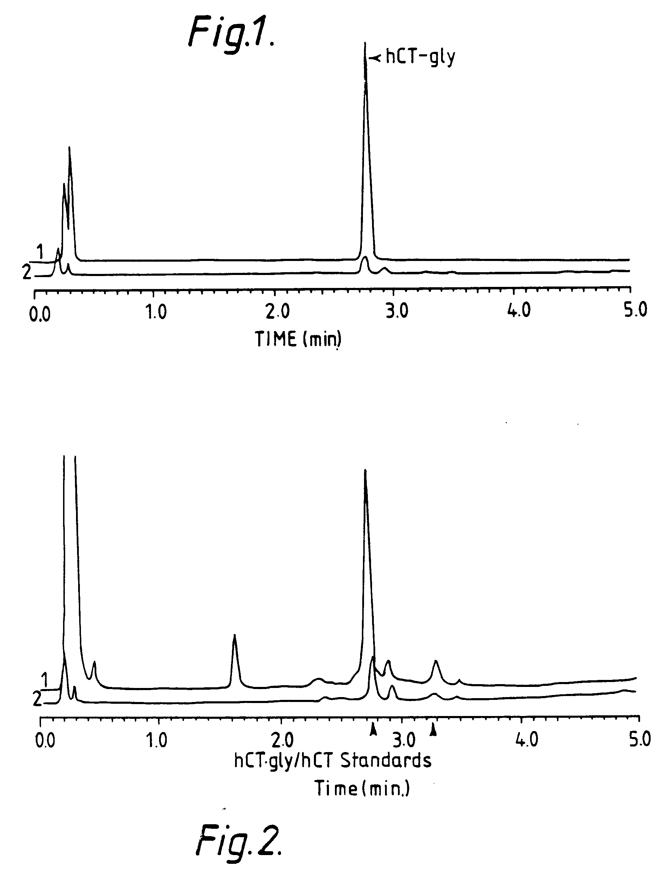

5 ul aliquots of the reaction mixture were taken at 0 and 420 minutes. The aliquots were analysed using HPLC. Figure 1 shows the results of these experiment and indicates clearly the loss of > 90% human calcitonyl-glycine from the solution phase of the reaction mixture over this period.

Comparative Example B

In order to show that human calcitonyl-glycine was in fact precipitated, the experiment described in Comparative Example A was repeated, and at the end of the reaction (after approximately 540 minutes) the remaining total sample (43 ul) was centrifuged (20,000 g x 5 minutes). The resulting pellet was dissolved in 50 ul of glacial acetic acid and a 10 ul aliquot was analysed by HPLC. In addition a 10 ul aliquot of the supernatant after centrifugation was analysed by HPLC. The results of the experiment are shown in Figure 2, and indicate that the majority (71%) of human calcitonyl-glycine was present in the pellet, indicating substantial precipitation of human calcitonyl-glycine.

Comparative Example C

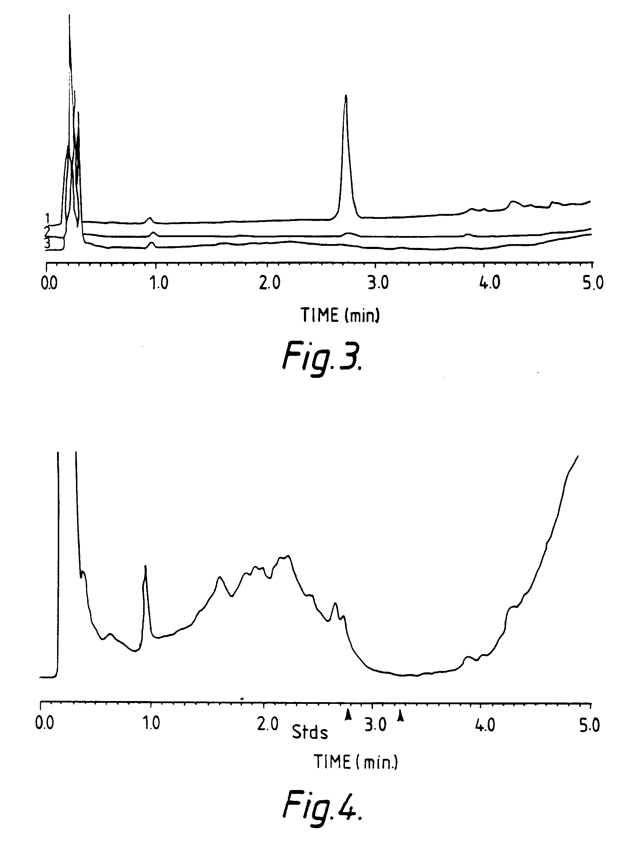

An experiment was conducted in order to show the precipitation of human calcitonyl-glycine in the presence and absence of amidation cofactors. Human calcitonyl-glycine was incubated at 37°C overnight in a buffer comprising 10 mM PIPES (pH 6.8) and 300 mM NaCl as a control, and in the same buffer containing additionally 0.3 mM Cu/EDTA and 3 mM ascorbic acid, each at a total volume, of 26 ul. Aliquots were centrifuged (20,000 g x 5 minutes) and analysed by HPLC. The results of the experiment are shown in Figure 3 in which Trace 1 shows 2.8 ul of the reaction mixture immediately after preparation and indicates a large peak showing the presence of human calcitonyl-glycine in the solution phase of the mixture. Trace 2 (7.5 ul) is the trace after an overnight incubation in the absence of Cu/EDTA and ascorbic acid and Trace 3 (7.5 ul) shows the trace again after an overnight incubation in the presence of Cu/EDTA and ascorbic acid.

Figure 4 shows a trace run at increased sensitivity of Trace 3 shown in Figure 3. The trace clearly shows the presence of complex multiple peaks with retention times between 2.5 and 3.0 minutes. The complex multiple peaks are taken to be polymerisation products of human calcitonyl-glycine.

Example 1

An experiment was performed to S-sulphonate human calcitonyl-glycine and human calcitonin.

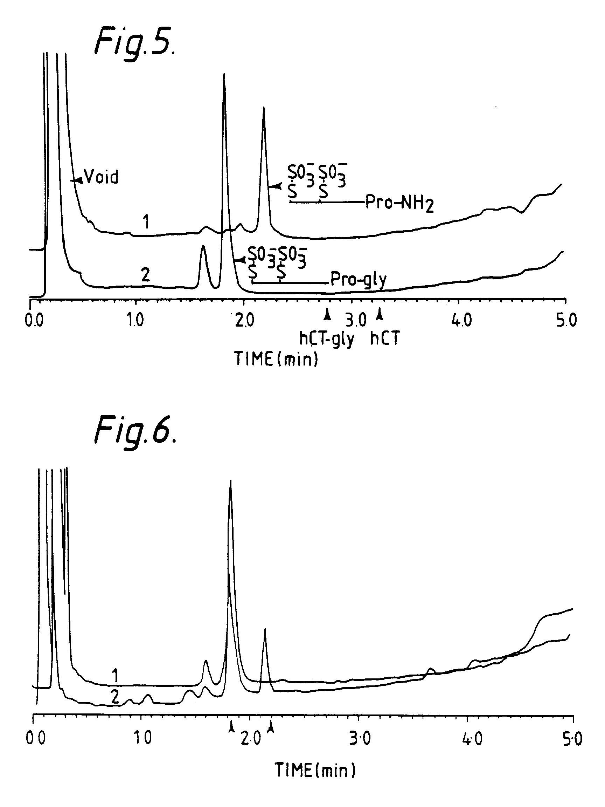

50 ul of 0.1 M $NaHCO_3$ (pH 9.3) was added to 100 ul of 2.5 mg/ml human calcitonyl-glycine in water. Additions of 1M $Na_2SO_3$ (13 ul) and 0.01 M $CuSO_4$ (3 ul) were made and the reaction mixture was left at room temperature for 60 minutes. The reaction was judged to be completed by the disappearance of all the original human calcitonyl-glycine (retention time approximately 2.75 minutes) and the appearance of the new sulphonated derivative (retention time approximately 1.8 minutes). The relevant HPLC traces are shown in Figure 5. The upper trace shows sulphonated human calcitonin, which was treated in a similar manner.

Example 2

An experiment was conducted to show the stabilisation afforded to human calcitonyl-glycine by S-sulphonation.

S-sulphonated human calcitonyl-glycine was incubated at 37°C with a porcine amidating enzyme preparation as described in Comparative Example A. Aliquots of the reaction mixture were taken, centrifuged (20,000 g x 5 minutes) and analysed by HPLC. Figure 6 shows the resulting trace which includes a new peak (30% yield) co-migrating with S-sulphonated human calcitonin (retention time approximately 2.5 minutes) after four hours of treatment with the amidating preparation. There is no precipitation of substrate or product as judged by the initial and final peak area.

The specific details of the HPLC determinations carried out in the above experiments were as follows:

Column:　　　Hypersil ODS 10 x 0.2 cm
% A :　　　　10 mM $NH_4HCO_3$
% B :　　　　$CH_3CN$
Gradient:　　15-50% B over 5 Min.
Flow Rate:　 1 ml/Min.
Detection:

| Fig. 1 | 1.1 AUFS, | $A_{225}$ |
| Fig. 2 | 1.5 AUFS, | $A_{225}$ |
| Fig. 3 | 0.2 AUFS, | $A_{225}$ |
| Fig. 4 | 0.025 AUFS, | $A_{225}$ |
| Fig. 5 & 6 | 0.05 AUFS, | $A_{225}$ |

Example 3

An experiment was carried out to thiophosphorylate hCT-gly and to determine the effect of varying experimental conditions.

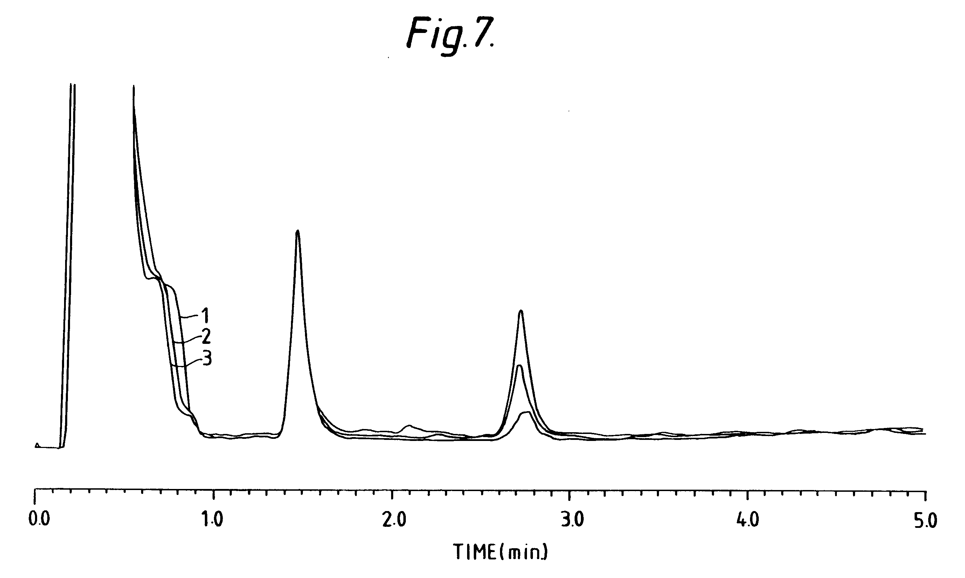

40 ul of 0.5M $Na_3O_3$ PS and 10 ul of 100 mM Cu/EDTA solution were added to 100 ul of a 0.6 mg/ml solution of hCT-gly which had been dried down. A final volume of 200 ul was obtained by adding 150 ul of 100 mM Tris buffer. Samples of 20 ul were analysed after reaction times of 30 minutes, 90 minutes and 150 minutes. As can be seen from the trace [Fig. 7] the product did not revert back to the starting material with time, but remained steady.

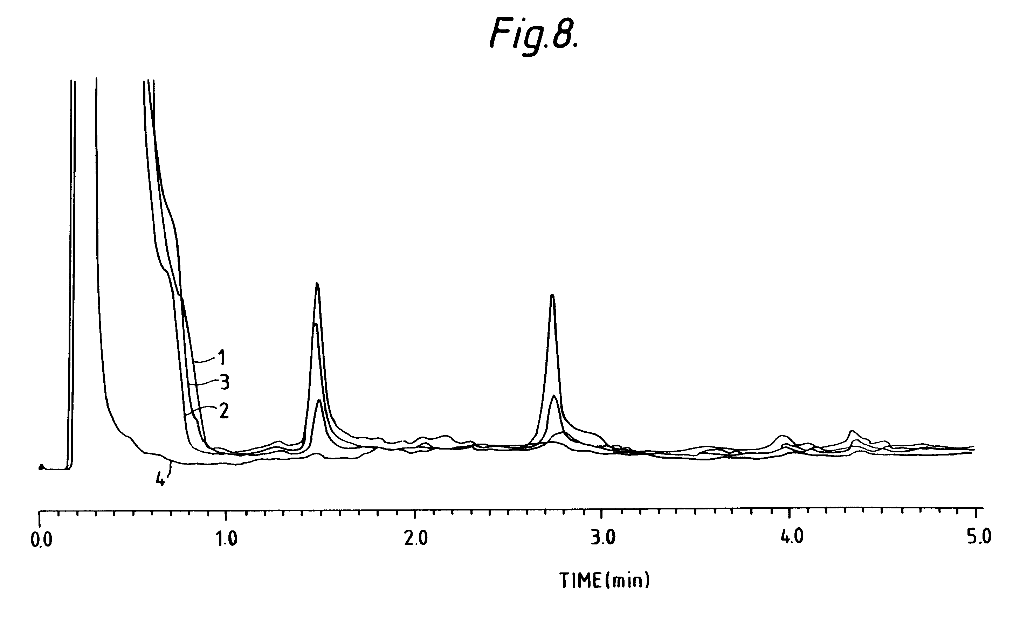

The reaction was repeated, using 10 ul of 10 mM Cu/EDTA solution instead of 10 ul of 100 mM Cu/EDTA solution. This decrease in Cu/EDTA appeared to have little effect on the reaction. A control was also carried out i.e.; reaction mixture minus $Na_3O_3$PS and as can be seen from the trace [Fig 8) the reaction has gone to completion after 100 minutes.

In the next experiment, the amount of $Na_3O_3$PS added was decreased i.e 10 mM final $Na_3O_3$PS instead of 100mM final $Na_3O_3$ PS. Like the decrease in Cu/EDTA, this appears to have had no great effect on the reaction [Fig. 9]. From these last two experiments it can be said that cupric ions are definitely required in order for the reaction to take place and that varying the amounts of Cu/EDTA and $Na_3O_3$PS has had very little effect on the reaction.

The thiophosphate protected hCT-gly is then amidated substantially as described in Example 2.

The specific details of the HPLC determinations are as described above in Example 2.

Example 4

SULPHONATION of hCGRP

A reaction mixture consisting of 14.4 mg of PENINSULAR, CRUDE CGRP; Tris (200 mM, 10 ml); and $Na_2SO_3$ (1M; 500ul); Cu/EDTA (10 mM; 100ul) was left to stand at room temperature and aliquots removed for injection onto Synchropak CM 300

A = HPLC water

B = 2M NaCl/20mM (P) pH 6.00

(i.e. 116.88g of NaCl in 1 litre HPLC $H_2O$ + 2ml $H_3PO_3$, pH - 6.00 with dilute NaOH)

C = MeCN

| T = 0 - 30%; | %B = 1 - 20%; | %C = 10 - 10 |
| T = 30 - 50%; | %B = 20 - 90%; | %C = 10 - 10 |

Flow = 3.0 ml min$^{-1}$; wavelength = 230 nm

The reaction was complete after 330 minutes. The peak at retention time 20.3 minutes was collected and freeze dried. The residue was dissolved in ca 3 ml 0.1m HOAC and loaded onto Sephadex G-10 for desalting. The column was eluted. The first peak was collected and freeze-dried. A sample of the resulting residue was sent for mass spectrum analysis.

The mass spectrum (shown in Figure 10) was run at acidic pH and gave a very weak protonated molecular ion in positive ion mode with a small fragment ion showing loss of one sulphate group 80 amu lower.

It will be understood that the present invention is described by way of example only and modification of details may be made within the scope of the invention.

**Claims**

1. A process for producing a first polypeptide having a C-terminal amide group, where the first polypeptide has one or more cysteine or cystine amino acid residues, comprising the step of reacting a second polypeptide comprising the amino acid sequence of the first polypeptide and a C-terminal glycine amino acid residue, with an amidating enzyme, characterised in that the cysteine or cystine sulphur group or groups is or are protected at least during the amidation step.

2. A process according to claim 1 wherein the cysteine or cystine sulphur group or groups of the second polypeptide is or are reversibly protected.

3. A process according to claim 1 wherein the cysteine or cystine sulphur group or groups of the second polypeptide is or are protected by a sulphite or a thiophosphate group.

4. A process according to claim 1 wherein the cysteine or cystine sulphur group or groups of the second polypeptide is or are protected by a charged protecting group.

5. A process according to any of the preceding claims wherein the first polypeptide is human calcitonin or human calcitonin gene-related peptide.

6. A polypeptide having one or more cysteine or cystine amino acid residues and a C-terminal glycine amino acid residue when used in a process according to claim 1 characterised in that the or each cysteine or cystine sulphur group or groups is or are protected.

7. A polypeptide according to claim 6 wherein the or each cysteine or cystine sulphur group or groups is or are protected by a sulphite or thiophosphate group.

8. A polypeptide according to claim 6 or claim 7 wherein the polypeptide is human calcitonin-gly or human calcitonin gene related peptide-gly.

9. A process according to Claim 1 wherein the amidating enzyme preparation includes a compound

7

EP 0 236 416 B1

capable of chelating copper.

**10.** A process according to Claim 9 wherein the compound capable of chelating copper is ethylenediaminetetraacetic acid.

## Revendications

**1.** Procédé de préparation d'un premier polypeptide ayant un groupe amide C-terminal, où le premier polypeptide a un ou plusieurs résidus amino acide de cystéine ou de cystine, comprenant l'étape de mise en réaction d'un deuxième polypeptide comprenant la séquence d'acides aminés du premier polypeptide et un résidu d'acide aminé de glycine C-terminal, avec une enzyme d'amidation, caractérisé en ce que le ou les groupes sulfures de la cystéine ou de la cystine est ou sont protégés au moins pendant l'étape d'amidation.

**2.** Procédé selon la revendication 1, dans lequel le ou les groupes sulfures de cystéine ou de cystine du deuxième polypeptide est ou sont protégés de façon réversible.

**3.** Procédé selon la revendication 1, dans lequel le ou les groupes sulfures de cystéine ou de cystine du deuxième polypeptide est ou sont protégés par un groupe sulfite ou thiophosphate.

**4.** Procédé selon la revendication 1, dans lequel le ou les groupes sulfures de la cystéine ou de la cystine du deuxième polypeptide est ou sont protégés par un groupe chargé de protection.

**5.** Procédé selon l'une quelconque des revendications précédentes, dans lequel le premier polypeptide est la calcitonine humaine ou un peptide apparenté au gène de la calcitonine humaine.

**6.** Polypeptide ayant un ou plusieurs résidus amino acide de cystéine ou de cystine et un résidu amino acide de glycine C-terminal quand il est utilisé dans un procédé selon la revendication 1, caractérisé en ce que le ou les ou chacun des groupes sulfures de la cystéine ou de la cystine est ou sont protégés.

**7.** Polypeptide selon la revendication 6, dans lequel le ou les ou chacun des groupes sulfures de la cystéine ou de la cystine est ou sont protégés par un groupe sulfite ou thiophosphate.

**8.** Polypeptide selon la revendication 6 ou 7, dans lequel le polypeptide est la calcitonine-gly humaine ou un peptide-gly apparenté au gène de la calcitonine humaine.

**9.** Procédé selon la revendication 1, dans lequel la préparation de l'enzyme d'amidation comprend un composé apte à complexer le cuivre.

**10.** Procédé selon la revendication 9, dans lequel le composé apte à complexer le cuivre est l'acide éthylènediaminetétraacétique.

## Patentansprüche

**1.** Verfahren zur Herstellung eines ersten Polypeptids mit einem C-terminalen Amid-Rest und ein oder mehr Cystein- oder Cystin-Aminosäureresten, bei dem ein zweites, die Aminosäuresequenz des ersten Polypeptids und einen C-terminalen Glycin-Aminosäurerest umfassendes Polypeptid mit einem Amidierungs-Enzym umgesetzt wird, dadurch gekennzeichnet, daß der oder die Cystein- oder Cystin-Schwefelreste zumindest während des Amidierungsschrittes geschützt sind.

**2.** Verfahren nach Anspruch 1, worin der oder die Cystein- oder Cystin-Schwefelreste des zweiten Polypeptids reversibel geschützt sind.

**3.** Verfahren noch Anspruch 1, worin der oder die Cystein- oder Cystin-Schwefelroste des zweiten Polypeptids durch einen Sulfit- oder Thiophosphat-Rest geschützt sind.

**4.** Verfahren nach Anspruch 1, worin der oder die Cystein- oder Cystin-Schwefelreste des zweiten Polypeptids durch eine geladene Schutzgruppe geschützt sind.

8

5. Verfahren nach einem der vorhergehenden Ansprüche, worin das erste Polypeptid humanes Calcitonin oder ein von humanem Calcitonin-Gen stammendes Peptid ist.

6. Polypeptid mit ein oder mehr Cystein- oder Cystin-Aminosäureresten und einem C-terminalen Glycin-Aminosäurerest in einem Verfahren nach Anspruch 1, dadurch gekennzeichnet, daß der oder alle Cystein- oder Cystin-Schwefelreste geschützt sind.

7. Polypeptid nach Anspruch 6, worin der oder alle Cystein- oder Cystin-Schwefelreste durch einen Sulfit- oder Thiophosphat-Rest geschützt sind.

8. Polypeptid noch Anspruch 6 oder 7, worin das Polypeptid humanes Calcitonin-Gly oder ein von humanem Calcitonin-Gen stammendes Peptid-Gly ist.

9. Verfahren nach Anspruch 1, worin die Amidierungs-Enzym-Preparation eine zur Kupferkomplexierung fähige Verbindung enthält.

10. Verfahren nach Anspruch 9, worin die zur Kupferkomplexierung fähige Verbindung Ethylendiamintetra-essigsäure ist.

*Fig.1.*

*Fig.2.*

TIME (min)

Fig.3.

TIME (min.)

Fig.4.

11

Fig.5.

Fig.6.

# Fig.7.

EP 0 236 416 B1

Fig.8.

EP 0 236 416 B1

TIME(min)

Fig. 9.

Fig. 10.